# EUROPEAN PATENT SPECIFICATION

(11) **EP 0 428 909 B1**
(45) Date of publication and mention of the grant of the patent: **15.12.1993**
(21) Application number: 90120626.8
(22) Date of filing: 27.10.1990
(51) Int. Cl.: C12M 1/28

(54) **Device for preserving samples and for isolating germs contained in said samples for the purposes of microbiological examination**
Vorrichtung zur Aufbewahrung von Proben und zur Isolierung von in diesen Proben enthaltenen Keimen für mikrobiologische Untersuchungszwecke
Appareil pour conserver des échantillons et pour isoler les germes contenus dans ces échantillons à des fins d'examen microbiologique

(30) Priority: 21.11.1989 IT 2246089
(43) Date of publication of application: 29.05.1991
(73) Proprietor: DIESSE DIAGNOSTICA SENESE s.r.l., I-20123 Milano (IT)
(72) Inventor: Ricci, Antonio, I-53035 Monteriggioni, Siena (IT)
(74) Representative: Giambrocono, Alfonso, Dr. Ing.

(56) References cited:
- FR-A- 2 057 723
- FR-A- 2 612 297

## Description

This industrial invention patent relates to a device for preserving samples and for isolating germs contained in said samples for the purposes of microbiological examination. More particularly, the device of the present invention enables the presumptive identification and quantitative evaluation of the bacterial load contained in said samples, without handling the sample.

Examples of samples are feces, urine, expectoration, tonsillar, nasal and ear swabs, and other material withdrawn for diagnostic purposes.

For a better understanding of the invention the present description will refer specifically to feces and urine examinations, however as stated the device can be used for the microbiological examination of various samples.

In the specific case of urine and feces, sterile plastic containers are currently marketed into which the patent introduces the feces or urine to be delivered to the laboratory for the necessary examination.

In the case of feces the sample must be delivered to the laboratory within a short time to prevent the sensitive pathogenic germs (such as Salmonella, Shigella, Yersinia enterocolitica and Staphylococcus) dying as a result of temperature or humidity decrease and thus not multiplying to allow the "isolation" stage to be proceeded with, and which precedes the "identification" stage. This latter is not involved in the present invention as it is conducted by known equipment.

In the case of urine the sample must also be delivered to the laboratory within a short time to prevent the germs contained in it, such as any contained E. coli, from developing and increasing in number, so that a normal urine sample can appear pathologic on examination, this being an obvious problem which must not be undervalued.

In consideration of the aforesaid concerning the non-limiting case of feces and urine, the object of the present invention is to provide a device which enables the bacterial state of a sample to be maintained constant for a time sufficient for its delivery to the laboratory, and subsequently any pathogens to be isolated without handling the sample.

This and further objects of the invention will be apparent to the expert of the art on reading the ensuing description.

The device according to the invention is essentially characterised by comprising a first aseptic enclosure for containing a biological liquid for in one case the development and in another case the maintaining of germs of interest, an element for introducing the sample to be examined into said biological liquid, an element carrying at least one selective culture medium and insertable into said aseptic enclosure, and a member provided with a suitable structure slidable with light contact along said selective culture medium for forming the distribution gradient of said biological liquid on said medium.

The device is illustrated by way of non-limiting example in the figures of the accompanying drawing, in which:
Figure 1 is a longitudinal section through the device arranged for feces;
Figure 2 is a longitudinal section through the device arranged for urine;
Figure 3 is a side view of the seeding element, usable both in the device arranged for feces and in the device arranged for urine;
Figure 4 is a section on the line IV-IV of Figure 3.

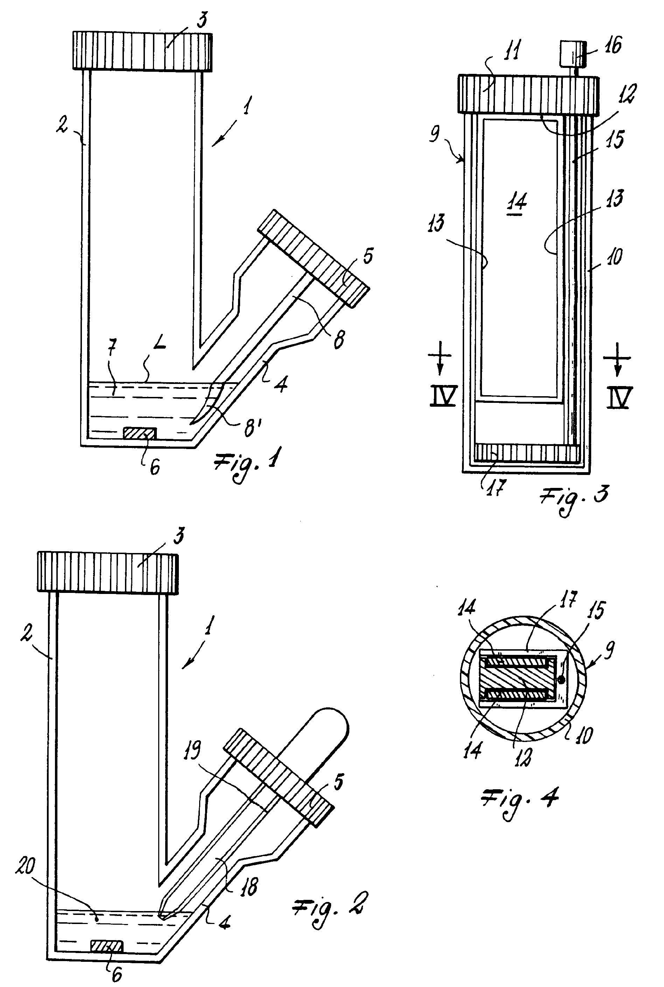

With reference to said figures and in particular Figures 1 to 3, the device consists of an aseptic body indicated overall by 1 and comprising a first enclosure 2 closable by a cap or stopper 3, and a second enclosure 4 inclined to the first enclosure 2 and opening into the lower part of this latter, it also being closable by a cap or stopper 5, the body being advantageously of transparent plastics.

On the inner side of the base of the first enclosure 2 there is provided a magnetic armature 6, said base containing a quantity of enrichment medium or broth 7, the level of which is indicated by L.

On the inner side of the cap 5 there is centrally provided a spatula 8, the end part 8′ of which is immersed in the culture medium or broth 7 when the cap 5 is in position.

The seeding element, indicated overall by 9, comprises an aseptic body 10 having its upper end closable by a cap or stopper 11.

There is provided rigid with the cap 11 a member 12 which in the illustrated case is of blade shape and is of such a length that its lower end touches the base of the first enclosure 2 when the cap or stopper 11 is in position and the body 10 has been inserted totally into the first enclosure 2.

In each of the opposing faces of the member 12 there is provided at least one pocket 13 for containing a suitable selective culture medium 14. The length of this pocket must be such that its lower end does not touch the level L of the enrichment medium or broth. In the cap or stopper 11 there is provided in a lateral position a hole for the passage of a rod 15, on the top of which, emerging from said cap or stopper, there is provided an operating knob 16, and at the lower end of which there is provided a fork 17, said rod being of such a length that when the cap or stopper 11 is in position and the body 10 has been completely inserted into the first enclosure 2, the fork 17 is immersed in the enrichment medium or broth 7, said fork 17 being of such a shape that its arms adhere against said selective culture medium 14 with slight pressure.

For feces analysis, the patient or subject concerned withdraws a lump of feces (about 1 gram) with the spatula 8 and insets it into the enrichment medium or broth 7 with the cap or stopper 3 of the first enclosure 2 kept closed, after which he closes the cap or stopper 5 of the second enclosure. Having done this he delivers the device 1 to the laboratory without any need for particular hurry as the enrichment broth 7 maintains, if not multiplies, the level of germs present in the feces to be examined. In the laboratory the technician removes the cap or stopper 3 of the first enclosure 2 and inserts into this the element 12 complete with cap or stopper 11. In this manner the fork 17 dips into the broth 7. Using a magnetic agitator the armature 6 is agitated to uniformly disperse the solid sample in the broth, after which it is allowed to incubate at 35°C for a time sufficient for any bacteria present to develop, after which said rod 15 is raised by the knob 16, so that said fork, now wetted with broth containing the germs, slides along the selective culture medium 14 provided on the member 12 to create a film with the gradient of the broth on said selective medium reducing from the bottom upwards, to effect in this manner the first stage of the examination, known as the "isolation" stage, which is then followed by the second or "identification" stage which as stated in the introduction to this description does not concern the present invention.

It is apparent from the aforegoing that the proposed invention has considerable advantages over the conventional system in that:
1) The patient limits the sample to the quantity necessary for the examination, so that as currently happens the laboratory technician does not have to select his required quantity from an excessive mass of feces;
2) Because of the presence of the enrichment broth 7 in the device 1 the lump of feces maintains the germs contained in it for a certain length of time, so that the subsequent "isolation" examination is without doubt more accurate and the time within which the sample need be delivered to the laboratory can be extended;
3) The laboratory technician totally avoids any unpleasant and infection-provoking contact with the feces, in that seeding is done by simply raising the rod 15 to which the seeding fork 17 is connected; and
4) The isolation stage is effected in a short time and if desired can be done by a simple machine which firstly homogenizes the feces by virtue of the presence of the magnetic armature 6 in the device 1, then incubates the sample at 35°C for the necessary time (generally 18⁴) to allow development of the germs of interest and their isolation by raising the rod 15 and fork 17, these all being operations which can be performed automatically in succession on several devices 1 all mounted on the same machine. In the case of urine, instead of the spatula 8, the device 1 comprises a dropper 18 with markings 19 to indicate the correct urine quantity required for analysis, and the first enclosure instead of containing enrichment broth 7 contains a suitable quantity of NaCl - polyvinylpyrrolidone or a likewise suitable solution such as boric acid, which as is well known blocks the development of any germs present while keeping them alive.

Isolation is done as described for feces, by using the element 9. In the case of other body fluids such as expectoration or various swabs, the spatula 8 or dropper 18 is replaced by a suitable implement able to retain a flock of cotton wool or gauze for insertion into the biological liquid for development or maintenance, the actual seeding being done as heretofore described.

The member 12, which in the illustrated embodiment is in the shape of a blade with two faces, can have any other cross-section and two or more faces, the fork 17 then being shaped to adapt to this cross-section. The cross-section of the enclosures 2 and 3 can also vary according to requirements.

Finally it should be noted that the device 1 could be marketed through normal channels (pharmacies) suitably packaged, and the seeding element 9 be made available to appropriate laboratories.

## Claims

1. A device for preserving samples and for isolating germs contained in said samples for the purposes of microbiological examination, characterised by comprising a first aseptic enclosure (2) for containing a biological liquid (7, 20) for in one case the development and in another case the maintaining of germs of interest, an, element (8, 18) for introducing the sample to be examined into said biological liquid, an element (9) carrying at least one selective culture medium (14) and insertable into said aseptic enclosure (2), and a rod (15) provided with a fork (17) slidable with light contact along said selective culture medium for forming the distribution gradient of said biological liquid on said medium.

2. A device as claimed in claim 1, characterised in that there is associated with said first aseptic enclosure (2) a second aseptic enclosure (4) for containing said element (8, 18) for introducing the sample to be examined into said biological liquid.

3. A device as claimed in claim 2, characterised in that for the examination of feces or other solid substances said element (8) for introducing the sample into said biological liquid is a spatula, spoon or the like, of which the end carrying the sample is immersed in said biological liquid.

4. A device as claimed in claim 2, characterised in that for the examination of liquids said element (18) for introducing the sample into said biological liquid is a dropper or similar dispenser, or a rod with cotton wool or gauze at its end.

5. A device as claimed in claim 1, characterised in that said element (9) carrying at least one selective culture medium (14) consists of an aseptic body (10), said element comprising a cap or stopper (11) with which said member (12) provided with the fork (17) is rigid.

6. A device as claimed in claim 5, characterised in that said member (12) is of elongate form with at least two faces in which pockets (13) are provided for containing said selective culture medium (14).

7. A device as claimed in claim 1 and one or more of the preceding claims, characterised in that said seeding structure consists of a rod (15) slidable within a hole provided in said cap or stopper (11), and a fork (17) rigid with the bottom of said rod an shaped to act with slight pressure against the selective culture medium (14) provided on said at least two faces of said member (12), that end of said rod distant from said fork emerging freely from said cap or stopper, the total length of said rod corresponding substantially to the total height of said first aseptic enclosure (2).

8. A device as claimed in claim 7, characterised in that that end of the rod (15) emerging from said cap or stopper (11) comprises means (16) for lifting and lowering the rod.

9. A device as claimed in claim 1, characterised by comprising means (6) for homogenizing the sample under examination in the biological liquid.

10. A device as claimed in claim 1, characterised in that the first aseptic enclosure (2) is also provided with a cap or stops (3).

11. A device as claimed in the preceding claims, characterised in that said element (8, 18) for introducing the sample into said biological liquid (7, 20) is rigid with a cap or stopper (5) of the second aseptic enclosure (4).

## Patentansprüche

1. Vorrichtung zur Aufbewahrung von Proben und zur Isolierung von in diesen Proben enthaltenen Keimen für mikrobiologische Untersuchungszwecke, **gekennzeichnet** durch ein erstes keimfreies Gehäuse (2) zur Aufnahme einer biologischen Flüssigkeit (7, 20), in einem Fall für die Entwicklung und im anderen Fall für das Bewahren von interessierenden Keimen, ein Element (8, 18) zum Einführen der zu prüfenden Probe in diese biologische Flüssigkeit, ein Element (9), das mindestens ein Kultursubstrat (14) trägt und in das genannte keimfreie Gehäuse (2) einsetzbar ist, und eine Stange (15), die mit einer Gabel (17) versehen ist, die unter leichter Berührung entlang dem ausgewählten Kultursubstrat gleitbar ist, um den Verteilungsgradient der genannten biologischen Flüssigkeit auf dem genannten Substrat zu bilden.

2. Vorrichtung nach Anspruch 1, **dadurch gekennzeichnet,** daß dem ersten keimfreien Gehäuse (2) ein zweites keimfreies Gehäuse (4) zur Aufnahme des genannten Elementes (8, 18) für die Einführung der zu prüfenden Probe in die biologische Flüssigkeit zugeordnet ist.

3. Vorrichtung nach Anspruch 2, **dadurch gekennzeichnet**, daß für die Prüfung von Exkrementen oder anderen festen Substanzen das Element (8) für die Einführung der Probe in die biologische Flüssigkeit ein Spatel, Löffel od. dgl. ist, dessen die Probe tragendes Ende in die biologische Flüssigkeit eintaucht.

4. Vorrichtung nach Anspruch 2, **dadurch gekennzeichnet,** daß für die Prüfung von Flüssigkeiten das Element (18) für die Einführung der Probe in die biologische Flüssigkeit ein Tropfer oder eine ähnliche Ausgabevorrichtung, oder eine Stange mit Baumwolle oder Gaze an ihrem Ende ist.

5. Vorrichtung nach Anspruch 1, **dadurch gekennzeichnet**, daß das mindestens ein ausgewähltes Kultursubstrat tragende Element (9) aus einem keimfreien Körper (10) besteht, wobei dieses Element eine Kappe oder einen Pfropfen (11) aufweist, mit der bzw. dem der mit der Gabel (17) versehene Teil (12) fest verbunden ist.

6. Vorrichtung nach Anspruch 5, **dadurch gekennzeichnet**, daß der genannte Teil (12) eine längliche Form mit mindestens zwei Seiten aufweist, in denen Taschen (13) zur Aufnahme des ausgewählten Kultursubstrates (14) vorgesehen sind.

7. Vorrichtung nach Anspruch 1 und einem oder mehreren der vorstehenden Ansprüche, **dadurch gekennzeichnet,** daß die genannte Impfanordnung aus einer Stange (15) besteht, die in einer in der Kappe oder dem Pfropfen (11) vorgesehenen Öffnung verschiebbar ist, sowie einer Gabel (17), die fest mit dem unteren Ende dieser Stange verbunden und so ausgebildet ist, daß sie mit einer leichten Pressung gegen das ausgewählte Kultursubstrat (14) wirkt, das an mindestens zwei Seiten des genannten Teiles (12) vorgesehen ist, wobei das der Gabel gegenüberliegende Ende der Stange aus der Kappe oder dem Pfropfen frei ausragt und die gesamte Lange der Stange im wesentlichen der Gesamthöhe des ersten keimfreien Gehäuses (2) entspricht.

8. Vorrichtung nach Anspruch 7, **dadurch gekennzeichnet**, daß das aus der Kappe oder dem Pfropfen (11) ausragende Ende der Stange (15) mit Mitteln (16) zum Heben und Senken der Stange versehen ist.

9. Vorrichtung nach Anspruch 1, **gekennzeichnet** durch Organe (6) zur Homogenisierung der Probe bei Prüfung in der biologischen Flüssigkeit.

10. Vorrichtung nach Anspruch 1, **dadurch gekennzeichnet**, daß das erste keimfreie Gehäuse (2) ebenfalls mit einer Kappe oder einem Pfropfen (3) versehen ist.

11. Vorrichtung nach einem der vorstehenden Ansprüche, **dadurch gekennzeichnet**, daß die Elemente (8, 18) für die Einführung der Probe in die biologische Flüssigkeit (7, 20) fest mit einer Kappe oder einem Pfropfen (5) des zweiten keimfreien Gehäuses (4) verbunden sind.

## Revendications

1. Dispositif permettant de conserver des échantillons et d'isoler les germes contenus dans lesdits échantillons aux fins d'analyse microbiologique, caractérisé par une première enceinte aseptique (2) destinée à recevoir un liquide biologique (7, 20) pour, d'une part, le développement et, d'autre part, la conservation des germes intéressants, un élément (8, 18) permettant d'introduire dans ledit liquide biologique l'échantillon à analyser, un élément (9) contenant au moins un milieu de culture sélectif (14) et s'adaptant à l'intérieur de ladite enceinte aseptique (2), et une tige (15) équipée d'une fourche (17) coulissant par léger contact le long dudit milieu de culture sélectif pour produire le gradient de répartition dudit liquide biologique sur ledit milieu.

2. Dispositif selon la revendication 1, caractérisé par l'existence d'une seconde chambre aseptique (4), associée à ladite première chambre aseptique (2), destinée à contenir ledit élément (8, 18) servant à introduire l'échantillon à examiner dans ledit liquide biologique.

3. Dispositif selon la revendication 2, caractérisé, pour l'examen d'échantillons de matières fécales ou autres substances solides, en ce que ledit élément (8) servant à introduire l'échantillon dans ledit liquide biologique est une spatule, une cuillère ou autre instrument similaire, dont l'extrémité contenant l'échantillon est immergée dans ledit liquide biologique.

4. Dispositif selon la revendication 2, caractérisé, pour l'examen de liquides, en ce que ledit élément (18) servant à introduire l'échantillon dans ledit liquide biologique est un compte-gouttes ou un autre instrument similaire servant à délivrer un liquide, ou une tige dotée à son extrémité d'un morceau de coton ou de gaze.

5. Dispositif selon la revendication 1, caractérisé en ce que ledit élément (9) contenant au moins un milieu de culture sélectif (14) se compose d'un corps aseptique (10), ledit élément comportant un couvercle ou bouchon (11) solidaire dudit membre (12) équipé de la fourche (17).

6. Dispositif selon la revendication 5, caractérisé en ce que ledit membre (12) est de forme allongée avec au moins deux faces contenant des poches (13) destinées à contenir ledit milieu de culture sélectif (14).

7. Dispositif selon la revendication 1 et l'une ou plusieurs des revendications précédentes, caractérisé en ce que ladite structure permettant d'ensemencer se compose d'une tige (15) coulissant dans un orifice percé dans ledit couvercle ou bouchon (11) et d'une fourche (17) solidaire de l'extrémité inférieure de ladite tige et permettant d'exercer une légère pression sur le milieu de culture sélectif (14) réparti sur les deux faces au moins dudit membre (12), l'extrémité de ladite tige opposée à ladite fourche dépassant librement à l'extérieur dudit couvercle ou bouchon, et la longueur totale de ladite tige correspondant essentiellement à la hauteur totale de ladite première enceinte aseptique (2).

8. Dispositif selon la revendication 7, caractérisé en ce que l'extrémité de la tige (15) dépassant à l'extérieur dudit couvercle ou bouchon (11) est équipée d'un moyen (16) permettant d'élever et d'abaisser la tige.

9. Dispositif selon la revendication 1, caractérisé en ce qu'il comprend un moyen (6) pour homogénéiser, dans le liquide biologique, l'échantillon à examiner.

10. Dispositif selon la revendication 1, caractérisé en ce que la première enceinte aseptique (2) est aussi équipée d'un couvercle ou bouchon (3).

11. Dispositif selon les revendications précédentes, caractérisé en ce que ledit élément (8, 18) servant à introduire l'échantillon dans ledit liquide biologique (7, 20) est solidaire du couvercle du bouchon (5) de la seconde enceinte aseptique (4).
